# EUROPEAN PATENT SPECIFICATION

(11) **EP 2 854 755 B1**
(45) Date of publication and mention of the grant of the patent: **27.11.2019**
(21) Application number: 13725539.4
(22) Date of filing: 20.05.2013
(51) Int. Cl.: A61K 8/81, A61K 8/23, A61Q 1/02, A61Q 7/02, A61K 8/891

(54) **COSMETIC PRODUCTS FOR REDUCING HAIR APPEARANCE**
KOSMETISCHE PRODUKTE ZUR BEHAARUNGSVERMINDERUNG
PRODUITS COSMÉTIQUES POUR RÉDUIRE L'APPARITION DE POILS

(30) Priority: 30.05.2012 US 201261652976 P
(43) Date of publication of application: 08.04.2015
(73) Proprietor: Noxell Corporation, Hunt Valley MD 21030-2098 (US)
(72) Inventor: TANAKA, Kojo, Ashiya Hyogo 659-0021 (JP); MITSUMATSU, Maya, Immunos 138648 (SG); SABINO, Michael, Christopher, Cincinnati, Ohio 45202 (US)
(74) Representative: Hanna Moore + Curley
(86) International application number: PCT/US2013/041823
(87) International publication number: WO 2013/181010

(56) References cited:
- EP-A1- 0 196 982
- EP-A1- 1 348 425
- WO-A1-01/12137
- WO-A1-2011/080743
- FR-A1- 2 794 765
- GB-A- 2 447 026
- US-A- 5 821 237
- US-A1- 2007 203 240

## Description

### FIELD OF THE INVENTION

The present invention relates to the reduction of hair appearance, and methods of use thereof.

### BACKGROUND OF THE INVENTION

Hair growth regulation often is desired to improve one's appearance and hygiene. Various methods and personal care products have been developed to remove unwanted hair, for example, shaving, electrolysis, waxing and depilatory creams. However, such conventional procedures frequently have drawbacks associated with them. Shaving, for instance, may cause nicks, cuts, rash and irritation and often leave undesirable stubble, as well as having to be carried out frequently. Electrolysis and laser hair removal can keep a treated area free of hair for prolonged periods of time, but can be either expensive, painful, and/or sometimes leave scarring. Waxing and plucking are painful and are poor options for shorter hair. Finally, depilatory creams, although effective, are messy to apply and typically are not recommended for frequent use due to their high irritancy potential. Therefore, these methods are not very welcomed by consumers, females especially, to managing unwanted facial hair though shaving is often used for facial hair removing. Shaving offers only short term hair removal, and must be repeated. For many consumers, continual growth of hair on the human scalp and face means that one must exert an on-going effort to hiding the hair that has grown out from the roots.

As an effort to mask skin defects such as spots and wrinkles, consumers tend to use make-up products such as foundations. Pigments are commonly formulated in make-up products for the purpose of providing natural finish yet having good coverage for minimizing the appearance of skin troubles, and seem to accentuate vellus hair appearance by pigment deposition.

The presence of unwanted facial hair is assumed to be one of the major yet unarticulated barriers to delivering this unmet need. It is therefore desirable to develop cosmetic compositions enabling consumers to manage unwanted hair, especially unwanted facial hair better.

### SUMMARY OF THE INVENTION

The present invention is the use of a cosmetic composition comprising: i) an organosiloxane resin in an amount from 0.3% to 10% by weight of the composition to provide hair appearance reducing effect on the skin, and ii) a dermatologically acceptable carrier for reducing hair appearance in the skin.

### BRIEF DESCRIPTION OF THE DRAWINGS

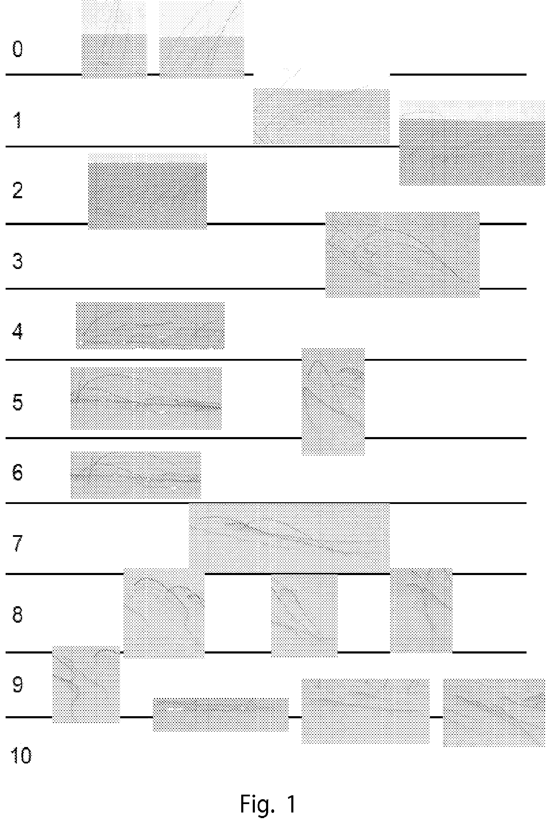

Fig. 1 is a photo of a grading sheet to rate hair lay down degree of hair.

### DETAILED DESCRIPTION OF THE INVENTION

The present invention uses compositions for providing reduced hair appearance in the skin, according to claim 1.

In all embodiments of the present invention, all percentages are by weight of the total composition, unless specifically stated otherwise. All percentages and ratios used herein are by weight of the total composition, unless otherwise designated. All ranges are inclusive and combinable. The number of significant digits conveys neither limitations on the indicated amounts nor on the accuracy of the measurements. All numerical amounts are understood to be modified by the word "about" unless otherwise specifically indicated. All measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

"Dermatologically acceptable carrier", as used herein, means that the carrier is suitable for topical application to the skin, has good aesthetic properties, is compatible with the actives of the present invention and any other components, and will not cause any safety or toxicity concerns.

"Derivatives," as used herein, includes but is not limited to, amide, ether, ester, amino, carboxyl, acetyl, and/or alcohol derivatives of a given compound.

"Hair," as used herein, includes hair on any part of the body.

"Hair growth regulation compound," as used herein, means a compound useful for regulating hair growth, and is understood not to encompass compounds such as depilatories.

"Hair lay down," as used herein, means decrease of the angle of hair standing.

"Isomers," as used herein, is understood to include D-isomers, L-isomers, and/or DL-isomers of a given compound.

"Keratinous tissue," as used herein, means to keratin-containing layers disposed as the outermost protective covering of mammals (e.g., humans, dogs, cats, etc.) which includes, but is not limited to, skin, hair, etc.

"Regulating hair growth," as used herein, means reducing, modulating, inhibiting, attenuating, retarding, and/or diminishing hair growth, and further may include regulating hair appearance, including maintaining a desired hair style for a longer period of time than would be expected without the use of a composition of the present invention. All relative terms used in connection with regulating hair growth and other benefits (such as softer, finer, reducing, less-noticeable, finer, etc.) are understood to mean that the benefit observed is relative to that which is observed or would be expected without the use of a composition described herein.

"Salts," as used herein, includes but is not limited to sodium, potassium, calcium, ammonium, manganese, copper, and/or magnesium salts of a given compound.

The compositions suitable for the present invention may take a variety of final forms, non-limiting examples of which include lotions, creams, liquids, gels, mousses, emulsions, multi-phase emulsions, pastes, semi-solid forms, and tonics, and may be applied to the skin via a variety of means.

The following describe some non-limiting embodiments of the present invention.

In one embodiment, the composition for the present invention further comprises one or more polyols.

In another embodiment, the compositions used for the present further comprise one or more useful actives for enhancing the benefit(s) of the hair growth regulation which is selected from the group consisting of reducing shaving frequency, improving the ease of hair removal, increasing shaving efficiency, making hair softer and finer, making hair less noticeable, slowing the re-growth of hair, reducing erythema and irritation to skin, maintaining a desired hair style for a longer period of time, maintaining a desired facial hair style for a longer period of time, reducing the appearance of pore size, and combinations thereof.

In another embodiment, the composition for the present invention further comprise one or more chronic skin care actives, providing at least one additional benefit to the skin. All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

In another embodiment, the composition for the present invention is a leave on product.

In another embodiment, the composition for the present invention is a make-up product such as foundations.

In another embodiment, the composition for the present invention is a personal care product such as body care products and skin care products.

### A. COMPOSITION

The compositions for the present invention, including the essential and optional components thereof, are described in detail hereinafter.

### Film Forming Polymer

The compositions used for the present invention comprise a film forming polymer. Without being limited by theory, it is believed that, by incorporating the film-forming polymers, the compositions can lay down hair during and after application of the compositions on the skin where the compositions are applied. Film forming polymers for the composition used for the present invention are those which can be used for skin, make-up and hair treatment compositions, and examples of suitable film forming polymeric materials are: organosiloxanes, including organosiloxane resins, fluid diorganopolysiloxane polymers and silicone ester waxes.

Examples of these polymers and cosmetic compositions containing them are found in PCT publication Nos. WO96/33689, published 10/31/96; WO97/17058, published 5/15/97; US Patent No. 5,505,937 issued to Castrogiovanni et al. 4/9/96, and PCT publication No. WO98/18431, published 5/7/98.

Examples of high molecular weight hydrocarbon polymers with viscosities of greater than about 50,000mPas include polybutene, polybutene terephthalate, polydecene, polycyclopentadiene, and similar linear and branched high molecular weight hydrocarbons. Preferred film forming polymers include organosiloxane resins comprising combinations of R₃SiO_{1/2} "M" units, R₂SiO "D" units, RSiO_{3/2} "T" units, SiO₂ "Q" units in ratios to each other that satisfy the relationship RₙSiO_{(4-n)/2} where n is a value between 1.0 and 1.50 and R is methyl. Note that a small amount, up to 5%, of silanol or alkoxy functionality may also be present in the resin structure as a result of processing. The organosiloxane resins must be solid at about 25°C and have a molecular weight range of from about 1,000 to about 10,000 grams/mole. The resin is soluble in organic solvents such as toluene, xylene, isoparaffins, and cyclosiloxanes or the volatile carrier, indicating that the resin is not sufficiently crosslinked such that the resin is insoluble in the volatile carrier. Particularly preferred are resins comprising repeating monofunctional or R₃SiO_{1/2} "M" units and the quadrofunctional or SiO₂ "Q" units, otherwise known as "MQ" resins as disclosed in U.S. Patent 5,330,747, Krzysik, issued July 19, 1994, incorporated herein by reference. In the present invention the ratio of the "M" to "Q" functional units is preferably about 0.7 and the value of n is 1.2. Organosiloxane resins such as these are commercially available such as trimethylsiloxysilicate / cyclomethicone D5 Blend available from GE Toshiba Silicone, Wacker 803 and 804 available from Wacker Silicones Corporation of Adrian Michigan, KP545 from Shin-Etsu Chemical and G. E. 1170-002 from the General Electric Company. In the present invention, by having film forming polymer mainly in the second layer, the film forming polymer will exist in a higher concentration at a localized area, and thereby forming a film of higher film intensity when applied to the skin, compared to the remainder of the composition. Such concentrated area of high film intensity provides improved adhesion of the entire composition to the skin. Namely, by providing the film forming polymer mainly in the second layer, the amount of film forming polymer included in the entire composition can be reduced, or if the same amount of film forming polymer is formulated in the second layer, an entire composition having improved adhesion is obtained.

A film forming organosiloxane resin in the compositions of the present invention is present in an amount from 0.3% to 10% by weight of the composition to provide hair appearance reducing effect on the skin, and the amount can be determined without undue experiment considering characteristics of a film forming polymer such as film forming capacity and impacts on skin sensory feel. Amount of a film forming polymer in the present invention may be from about 0.3% to about 10%, more preferably from about 1% to about 6%, and more preferably 1.5% to about 5% by weight of the composition.

### Dermatologically Acceptable Carrier

The compositions for the present invention also comprise a dermatologically acceptable carrier. The dermatologically acceptable carrier may be present in an amount of from about 50% to about 99.3%, preferably from about 60% to about 99%, more preferably from about 70% to about 98.5% by weight of the composition.

The carrier can be in a wide variety of forms. For example, emulsion carriers, including, but not limited to, oil-in-water, water-in-oil, water-in-oil-in-water, oil-in-water-in-oil emulsions, aqueous solution, water gel, oil dispersion and oil gel are useful herein. Oil in the carrier herein is understood to include silicone oil.

To maximize hair lay-down performance of the present composition, dermatologically acceptable carrier may be a carrier having an aqueous phase as a continuation phase such as oil-in-water and wter-in-oil-in-water, aqueous solution, and water gel. However, dermatologically acceptable carrier may be selected different to consider other performance of the composition. The carrier may contain water in the range of from about 30% to about 80%, preferably from about 40% to about 60% by the weight of the composition.

### Optional Components

The compositions for the present invention may further contain additional components such as those conventionally used in topical products, e.g., for providing aesthetic or functional benefit to the composition or skin, such as sensory benefits relating to appearance, smell, or feel, therapeutic benefits, or prophylactic benefits (it is to be understood that the above-described required materials may themselves provide such benefits).

The CTFA Cosmetic Ingredient Handbook, Second Edition (1992) describes a wide variety of nonlimiting cosmetic and pharmaceutical ingredients commonly used in the industry, which are suitable for use in the topical compositions of the present invention. Such other materials may be dissolved or dispersed in the composition, depending on the relative solubilities of the components of the composition.

### 1. Polyol

The composition for the present invention may further comprise at least one polyol. Without being limited by theory, it is believed that polyols, in addition to its well known humectants function, are able to soften hair and thus hair becomes more susceptible for being laid down by a composition comprising a film forming polymer.

Polyols useful herein include polyhydric alcohols such as glycerin, 1,3-butylene glycol, propylene glycol, hexylene glycol, propane diol, ethylene glycol, diethylene glycol, dipropylene glycol, diglycerin, sorbitol, and other sugars which are in liquid form at ambient temperature. Also useful herein are water soluble alkoxylated nonionic polymers such as polyethylene glycol. Glycerine is a preferred.

The amount of polyols in the compositions for the present invention can be determined depending on the desired characteristics of the product without undue experiment. In one embodiment, the polyol may be preferably comprised by weight of the composition at from about from about 5% to about 50%, preferably from about 7% to about 30% by weight of the composition, preferably from about 10% to about 25%, by weight of the composition.

### 2. Hair Growth Regulation Compounds

The compositions for the present invention may further comprise from about 0.001% to about 10%, preferably from about 0.01% to about 5%, and more preferably from about 0. 1% to about 1% of at least one hair growth regulation compound by weight of the composition. Preferably, the hair growth regulation compound is selected from the group consisting of butylated hydroxytoluene, butylated hydroxyanisole, hexamidine compounds, cetyl pyridinium chloride, green tea catechins, phytosterols, ursolic acid, difluoromethylornithine, alpha-methylornithine, 1,8-diaminooctane, bathocuprione, alanosine, arginine, extracts derived from soy plants, cinnamic acid, cinnamyl anthranilate, mannosamine and salts, derivatives and mixtures of any of the foregoing. As used herein, "hexamidine" includes hexamidine salts, any isomers and tautomers of such and is commercially available as hexamidine isethionate under the tradename Elastab® HP100 from Laboratoires Serobiologiques (Pulnoy, France).

The composition of the present invention may further comprise additional hair growth regulation compound selected from the group consisting of glyceryl dilaurate, including derivatives such as alpha, alpha-dilaurin and 1,2-dilauroyl-SN-glycerol apigenin, tetrahydrocurcumin, oleanolic acid, azelaic acid, sulforaphane, canavanine, pyridoxal-5-phosphate, phytic acid, tannic acid, grape seed extract, L-NAME, benzamidine, sodium butyrate, betulinic acid, polyornithine, polyarginine, fisetin, methyl-jasmonate, cis-jasmone caffeic acid phenethyl ester, delphinidin, ethyl abietate, esculetin, sorbic acid methyl ester, L-canaline, N-formyl-methionine, N-formyl-alanine, taurine, palmitoyl carnitine, undecanol, undecylenic acid, rutin, fusidic acid, phenyl pyruvic acid, L-isoleucine, phenyl glycine, silibinin, silymarin, L-ascorbic acid-6-palmitate, N-undecylenoyl-L-phenylalanine, and salts, derivatives and mixtures of any of the foregoing.

### 3. Pigments

### i) Soft focus powder

The composition for the present invention may further comprise at least one soft focus powder. Soft focus powder is a pigment that is particularly effective in providing a soft focus effect to the composition, namely natural finish yet having good coverage for minimizing the appearance of skin troubles, when incorporated in a defined amount.

The soft focus powder useful herein includes polymethyl/methacrylate (PMMA), silica, hybrid pigments such as alumina treated mica, titanium dioxide treated talc, titanium dioxide treated mica, vinyl dimethicone/methicone silsesquioxane crosspolymer, alumina, barium sulfate and synthetic mica.

Preferred content level of soft focus powder is from about 0.1% to about 10%, more preferably from about 1% to about 5% by weight of the composition.

### ii) Soft focus silicon elastomer

The composition for the present invention may further comprise at least one soft focus silicone elastomer. Soft focus silicone elastomer is crosslinked siloxane elastomer which is particularly effective in providing soft focus effect to the skin. In other words, when incorporated in a cosmetic product a defined amount of silicone elastomer, the silicone elastomer can provide natural finish yet having good coverage for minimizing the appearance of skin troubles. Silicone elastomer suitable for use herein can be emulsifying or non-emulsifying crosslinked siloxane elastomer or mixtures thereof. The term "non-emulsifying" as used herein, defines crosslinked organopolysiloxane elastomer from which polyoxyalkylene units are absent. The term "emulsifying" as used herein, means crosslinked organopolysiloxane elastomer having at least one polyoxyalkylene (e.g., polyoxyethylene or polyoxypropylene) unit. Non-emulsifying elastomer useful in the present invention is formed via crosslinking organohydroenpolysiloxane with an alpha, omega-diene. Emulsifying elastomer herein includes polyoxyalkylene modified elastomer formed via crosslinking from organohydrogenpolysiloxane with polyoxyalkylene diene or organohydrogenpolysiloxane containing at least one polyether group crosslinked with an alpha, omega-diene. Emulsifying crosslinked organopolysiloxane elastomer can notably be chosen from the crosslinked polymer described in US Patents 5,412,004, 5,837,793, and 5,811,487. In addition, an emulsifying elastomer comprised of dimethicone copolyol crosspolymer (and dimethicone) is available from Shin Etsu under the tradename KSG-21.

Preferred content level of silicone elastomer is from about 0.1% to about 10%, more preferably from about 1% to about 5% by weight of the composition.

### iii) Pigment powder (mention make-up product)

The composition for the present invention may comprise from about 2% to about 45%, preferably from about 5% to about 30% of a pigment powder by weight of the composition, especially when the composition is a make-up composition. The pigments included in the pigment powder component herein are typically hydrophobic in nature, or hydrophobically treated. By keeping the level of pigment component low, the entire composition maintains flexibility to accommodate other components which provide spreadability, moisturization, and fresh and light feel. The species and levels of the pigments are selected to provide, for example, shade, coverage, good wear performance, and stability in the composition.

Pigments useful for the pigment component herein are inorganic and organic powder such as talc, mica, sericite, silica, magnesium silicate, synthetic fluorphlogopite, calcium silicate, aluminum silicate, bentonite and montmorillonite; pearl pigments such as alumina, barium sulfate, calcium secondary phosphate, calcium carbonate, coverage titanium oxide, finely divided titanium oxide, zirconium oxide, normal particle size zinc oxide, hydroxy apatite, iron oxide, iron titanate, ultramarine blue, Prussian blue, chromium oxide, chromium hydroxide, cobalt oxide, cobalt titanate, titanium oxide coated mica; organic powder such as polyester, polyethylene, polystyrene, methyl methacrylate resin, cellulose, 12-nylon, 6-nylon, styrene-acrylic acid copolymers, polypropylene, vinyl chloride polymer, tetrafluoroethylene polymer, boron nitride, fish scale guanine, laked tar color dyes, and laked natural color dyes. Such pigments may be treated with a hydrophobical treatment agent, including: silicone such as methicone, dimethicone, and perfluoroalkylsilane; fatty material such as stearic acid and disodium hydrogenated glutamate; metal soap such as aluminium dimyristate; aluminium hydrogenated tallow glutamate, hydrogenated lecithin, lauroyl lysine, aluminium salt of perfluoroalkyl phosphate, and aluminium hydroxide as to reduce the activity for titanium dioxide, and mixtures thereof.

### 4. Skin Active Agent

The compositions for the present invention may comprise a safe and effective amount of a skin active agent. The term "skin active agent" as used herein, means an active ingredient which provides a cosmetic and/or therapeutic effect to the area of application on the skin, hair, or nails. The skin active agents useful herein include skin lightening agents, anti-acne agents, emollients, non-steroidal anti-inflammatory agents, topical anaesthetics, artificial tanning agents, antimicrobial and anti-fungal actives, skin soothing agents, sun screening agents, skin barrier repair agents, anti-wrinkle agents, anti-skin atrophy actives, lipids, sebum inhibitors, sebum inhibitors, skin sensates, protease inhibitors, skin tightening agents, anti-itch agents, hair growth inhibitors, desquamation enzyme enhancers, anti-glycation agents, and mixtures thereof. When included, the present composition comprises from about 0.001% to about 20%, preferably from about 0.1% to about 10% by weight of the composition of at least one skin active agent.

### 5. Sunscreen agent

The compositions for the present invention may comprise a safe and effective amount of sunscreen agent such as a UV protection powder and a UV absorbing agent.

UV protection powder has a particle size of less than 100nm, which size provide very little coverage effect to the skin. The composition of the present invention may comprise from about 0% to about 20%, preferably from about 0.1% to about 10% of a UV protection powder, such as micronized titanium dioxide and micronized zinc oxide. The powder included in the pigment component herein is typically hydrophobic in nature, or hydrophobically treated. Commercially available UV protection powder is titanium dioxide and methicone SI-TTO-S-3Z available from Miyoshi Kasei, titanium dioxide and dimethicone and aluminum hydroxide and stearic acid: SAST-UFTR-Z available from Miyoshi Kasei, Zinc oxide : Finex series available from Sakai Chemical Industry.

UV absorbing agent useful herein includes, for example, 2-ethylhexyl-p-methoxycinnamate (commercially available as PARSOL MCX), butylmethoxydibenzoyl-methane, 2-hydroxy-4-methoxybenzo-phenone, 2-phenylbenzimidazole-5-sulfonic acid, octyldimethyl-p-aminobenzoic acid, octocrylene, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, Eusolex™ 6300, Octocrylene, Avobenzone (commercially available as Parsol 1789), and mixtures thereof. A wide

When included, the present composition comprises from about 0.5% to about 20%, preferably from about 1% to about 15% by weight of the composition of a UV absorbing agent.

### Application of the composition

Application of the compositions can occur through a variety of means, including with the fingers or hands, or by using an implement. Non-limiting examples of implements include a pad, cotton ball, applicator pen, roll-on applicator, and spray applicator

The composition may be applied to any part of the skin in need of reducing hair appearance and/or any additional benefit described herein.

### Application Regimens

The compositions in the present invention may be applied to the skin sequentially with a skin care product or make-up product. As an example, a cosmetic composition comprising at least one hair growth regulation compound is applied on the skin, and then the composition in the present invention is applied to the skin, wherein the cosmetic composition and the composition in the present invention differ from each other in terms of their compositions. As another example, the composition in the present invention is applied to the skin, and then, a cosmetic composition comprising at least one hair growth regulation compound applied to the skin, wherein the cosmetic composition and the composition in the present invention differ from each other in terms of their compositions. The regimen suggested herein can meet consumers demands in both long term hair minimization and short term hair appearance reduction by employing a cosmetic composition comprising at least one hair growth regulation compound, and a composition comprising a film forming polymer, and it can eventually provide enhanced hair appearance reducing effect.

In one embodiment, a method in the present invention comprises a) applying to the skin a first composition comprising a film forming polymer in an amount sufficient to provide hair appearance reducing effect on the skin and dermatologically acceptable carrier; and b) applying to the skin a second cosmetic composition comprising at least one hair growth regulation compound and dermatologically acceptable carrier; and wherein the first and second compositions differ from each other in terms of their compositions. In the embodiment, the first composition is preferably applied along the direction of hair growth on the area of the skin.

In another embodiment, a method in the present invention comprises a) applying to the skin a first cosmetic composition comprising at least one hair growth regulation compound and dermatologically acceptable carrier; and b) applying to the skin a second composition comprising a film forming polymer in an amount sufficient to provide hair appearance reducing effect on the skin and dermatologically acceptable carrier; and wherein the first and second compositions differ from each other in terms of their compositions. In the embodiment, the second composition is preferably applied along the direction of hair growth on the area of the skin.

### TEST METHOD

### Hair Lay-Down Measurement

21 arm hairs with various lengths are implanted in artificial skin such as Bio Skin (model No.HO64-001) from Beaulax Co., Ltd. (Japan) Hair length is in the range of 0.5-1.8cm after implanted in the artificial skin. Excess hairs at the backside of the artificial skin are cut and glue such as cyanoacrylate type instant glue is applied to the backside to adhere hairs on the artificial skin. 0.0125g (0.0005g/cm2) of a test sample is applied on the Bio Skin by a finger with finger sack until the sample is evenly distributed. 5 min later, each hair is rated based on a grading sheet of Fig. 1. An average hair lay down rate is calculated by dividing total of rating numbers by total numbers of hair. The number of hairs and the amount of a sample can be adjusted.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

Cosmetic compositions were prepared by conventional methods from the following components.

| | Ex. 1 | Ex. 2 | Ex. 3 | Ex. 4 | Ex. 5 | Com 1 |
|---|---|---|---|---|---|---|
| KF-7312J *¹ | 6.000 | 4.000 | | | | |
| Luviskol K17 *² | | | 2.000 | | 0.500 | |
| Daitosol 5000 SJ *³ | | | | 5.000 | | |
| Glycerin USP | 10.000 | 5.000 | 10.000 | | 10.000 | 10.000 |
| Propylene glycol | | | | 30.000 | | |
| Pentylene glycol | | 2.500 | | 3.000 | | 2.500 |
| 1,2 HEXANEDIOL | | 0.500 | | 2.000 | | 0.500 |
| DI Water | 52.500 | 48.659 | 54.467 | 37.341 | 55.967 | 43.659 |
| SA TTC-30 *⁴ | | 4.500 | 2.000 | | 2.000 | 4.500 |
| TTC-30 *⁵ | | | | 5.000 | | |
| Cyclomethicone D5 | 15.0000 | 11.1010 | | | | 15.1010 |
| Tridecyl isononanoate (WHICKENOL 153) | 10.000 | 5.000 | 2.833 | | 2.833 | 5.000 |
| KF-6028 *⁶ | 2.000 | 1.500 | | | | 1.500 |
| Sorbitan isostearate (CRILL6) | 0.500 | 1.500 | | | | 1.500 |
| Brij 72 *⁷ | | | 0.100 | | 0.100 | |
| Brij S721 *⁸ | | | 0.900 | | 0.900 | |
| Polysorbate 20 | | | | 1.000 | | |
| Octyl methoxylcinnamate, USP (UVINUL MC80) | | 2.000 | 7.000 | | 7.000 | 2.000 |
| Tocopheryl acetate (DL) | | | 0.200 | | 0.200 | |
| Cetyl alcohol (APJ) | | | 0.200 | | 0.200 | |
| Stearyl alcohol (LEROL C18) | | | 0.600 | | 0.600 | |
| Behenyl alcohol (High stearyl) | | | 0.400 | | 0.400 | |
| BENTONE GEL VS-5PCV | | 1.500 | | | | 1.500 |
| RHEOPEARL KL2 *⁹ | 3.500 | 2.500 | | | | 2.500 |
| Ozokerite Wax | | | | | | |
| BHT | | 0.500 | | | | 0.500 |
| Ethyleparaben, NF | | | 0.200 | | 0.200 | |
| Propylparaben | | | 0.150 | | 0.150 | |
| SA/NAI-TR-10/D5 *¹⁰ | | 3.750 | | | | 3.750 |
| SA/NAI-Y-10/D5 *¹¹ | | 0.637 | | | | 0.637 |
| SA/NAI-R-10/D5 *¹² | | 0.327 | | | | 0.327 |
| SA/NAI-B-10/D5 *¹³ | | 0.196 | | | | 0.196 |
| SI-2 Yellow LL-100P *¹⁴ | | | 0.435 | | 0.435 | |
| SI-2 Red R-516P *¹⁵ | | | 0.240 | | 0.240 | |
| SI-2 Black BL-100P *¹⁶ | | | 0.096 | | 0.096 | |
| SA Titanium Dioxide CR-50 *¹⁷ | | | 5.544 | | 5.544 | |
| SI TALC *¹⁸ | | | 0.835 | | 0.835 | |
| SI SILDEX H-52 *¹⁹ | | 2.000 | | | | 2.000 |
| SI TALC CT-20 *²⁰ | | 2.000 | | | | 2.000 |
| Silica Pearl P-4 *²¹ | | | 10.000 | 5.000 | 10.000 | |
| PGSS-22 TiO2 R250 *²² | | | | 8.330 | | |
| PGSS-22 Yellow No.602P *²³ | | | | 0.620 | | |
| PGSS-22 Red No.211P Mix *²⁴ | | | | 0.404 | | |
| PGSS-22 Black No.710P *²⁵ | | | | 0.205 | | |
| SEPIGEL 305 *²⁶ | | | 1.000 | 1.400 | 1.000 | |
| MAKIMOUSSE 12 *²⁷ | | | | 0.250 | | |
| Hexamidine diisethionate | | 0.080 | | | | 0.080 |
| EDTA-2NA | 0.050 | | 0.050 | 0.050 | 0.050 | |
| Phenoxy Ethanol | 0.300 | 0.100 | | 0.400 | | 0.100 |
| Benzyl Alcohol | 0.150 | 0.150 | 0.500 | | 0.500 | 0.150 |
| Methylparaben | | | 0.250 | | 0.250 | |
| Total | 100.000 | 100.000 | 100.000 | 100.000 | 100.000 | 100.000 |

| | | | | | | |
|---|---|---|---|---|---|---|
| *1) Trimethylsiloxysilicate (50%) and Cyclopentasiloxane (50%) form Shin-Etsu Chemical Co. *2) PVP (100%) from BASF Corporation *3) Acrylates/Ethylhexyl Acrylate Copolymer (100%) from Daito Kasei Kogyo Co., Ltd. *4) Titanium Dioxide and Aluminum Hydroxide and Talc and Magnesium Stearate and Dimethicone from Miyoshi Kasei, Inc. *5) Titanium Dioxide and Aluminum Hydroxide and Talc and Magnesium Stearate from Miyoshi Kasei, Inc. *6) PEG-9 Polydimethylsiloxyethyl Dimethicone from Shin-Etsu Chemical Co. *7) Isosteareth-2 from Croda, Inc. *8) Steareth-21 from Croda, Inc. *9) Dextrin Palmitate from Chiba Flour Milling Company, Ltd. *10) Titanium Dioxide and Cyclomethicone and Dimethicone and Disodium Stearoyl Glutamate and Aluminum Hydroxide from Miyoshi Kasei, Inc. *11) Iron Oxides and Cyclomethicone and Dimethicone and Disodium Stearoyl Glutamate and Aluminum Hydroxide from Miyoshi Kasei, Inc. *12) Iron Oxides and Cyclomethicone and Dimethicone and Disodium Stearoyl Glutamate and Aluminum Hydroxide from Miyoshi Kasei, Inc. *13) Iron Oxides and Cyclomethicone and Dimethicone and Disodium Stearoyl Glutamate and Aluminum Hydroxide from Miyoshi Kasei, Inc. *14) Iron Oxides and Methicone from Daito Kasei Kogyo Co., Ltd. *15) Iron Oxides and Methicone from Daito Kasei Kogyo Co., Ltd. *16) Iron Oxides and Methicone from Daito Kasei Kogyo Co., Ltd. *17) Titanium Dioxide and Aluminum Hydroxide and Dimethicone from Miyoshi Kasei, Inc. *18) Talc and from Methicone from Miyoshi Kasei, Inc. *19) Silica and Methicone from Miyoshi Kasei, Inc. *20) Talc and from Methicone from Miyoshi Kasei, Inc. *21) Silica from Presperse LLC *22) Titanium Dioxide and Aluminum Hydroxide and Methoxy PEG-10 Propyltrimethoxysilane and Silica from Daito Kasei Kogyo Co., Ltd. *23) Iron Oxides and Methoxy PEG-10 Propyltrimethoxysilane and Silica from Daito Kasei Kogyo Co., Ltd. *24) Iron Oxides and Methoxy PEG-10 Propyltrimethoxysilane and Silica from Daito Kasei Kogyo Co., Ltd. *25) Iron Oxides and Methoxy PEG-10 Propyltrimethoxysilane and Silica from Daito Kasei Kogyo Co., Ltd. *26) Polyacrylamide and Water and C13-14 Isoparaffin and Laureth-7 from Seppic *27) Sodium Polyacrylate Starch from Daito Kasei Kogyo Co., Ltd. | | | | | | |

As for Examples 1-3 and Comparison Examples land 2, in a suitable vessel, all hydrophilic and water soluble components except a thickener (SEPIGEL 305 *26) were blended together, and mixed until all of the components were dissolved. In another vessel, all hydrophobic and oil soluble components except a thickener (RHEOPEARL KL2 *9) were blended, and mixed until all of the components were homogenized. Mix above hydrophilic and hydrophobic ingredients for emulsification. A thickener was added to the obtained emulsion, and the emulsion was gently mixed. When RHEOPEARL KL2 is a thickener, the emulsion was heated until 90C, then it was cooled down.

As for Example 4, in a suitable vessel, all hydrophilic and water soluble components except a thickener (SEPIGEL 305 *26 and MAKIMOUSSE 12 *27) were blended together, and mixed until all of the components were dissolved. Thickeners were added the mixture and the mixture was gently mixed.

### Measurement of Hair lay down

Select examples were tested according to Hair Lay-Down Measurement, and provided the following average hair lay down rate. In the test, each sample

| | Ex. 1 | Ex. 2 | Ex. 3 | Ex. 4 | Ex. 5 | Com. 1 |
|---|---|---|---|---|---|---|
| Hair Lay-Down with random application | 1.3 | 1.1 | 1.8 | 2.4 | | |
| Hair Lay-Down with the direction of hair growth application | 1.9 | 1.5 | 3.0 | 5.1 | 1.9 | 0.9 |

## Claims

1. The use of a cosmetic composition comprising: i) an organosiloxane resin in an amount from 0.3% to 10% by weight of the composition to provide hair appearance reducing effect on the skin, and ii) a dermatologically acceptable carrier for reducing hair appearance in the skin.

2. The use according to claim 1 wherein said composition further comprises a hair growth regulation compound, and wherein said hair growth regulation compound is selected from the group consisting of butylated hydroxytoluene, butylated hydroxyanisole, hexamidine compounds, cetyl pyridinium chloride, green tea catechins, phytosterols, ursolic acid, difluoromethylornithine, alpha-methylornithine, 1,8-diaminooctane, bathocuprione, alanosine, arginine, extracts derived from soy plants, cinnamic acid, cinnamyl anthranilate, mannosamine, and salts, derivatives and mixtures of any of the foregoing.

3. The use according to claim 1 or 2, wherein said hair growth regulation compound is selected from the group consisting of butylated hydroxytoluene, butylated hydroxyanisole, hexamidine, cetyl pyridinium chloride, ursolic acid, and combinations thereof.

4. The use according to any one of claims 1 - 3, wherein said composition further comprises a polyol, and wherein said polyol is in the range of from 5% to 50% by weight of the composition.

5. The use according to any one of claims 1 - 4, wherein said composition comprises water in the range of from 30% to 80% by weight of the composition.

6. The use according to any one of claims 1 - 5, wherein said composition is a leave on make-up product that further comprises a pigment and at least one skin active agent.

7. The use according to any of the preceding claims, wherein said composition is applied along the direction of hair growth on the area of the skin wherein hair appearance reduction is desired.

8. The use according to claim 7 wherein a second composition is applied to the skin, which comprises at least one hair growth regulation compound and dermatologically acceptable carrier; and wherein the first and second compositions differ from each other in terms of their compositions.

9. The use of claim 8, wherein the hair growth regulation compound of the second composition is selected from the group consisting of butylated hydroxytoluene, butylated hydroxyanisole, hexamidine, cetyl pyridinium chloride, ursolic acid, and combinations thereof.

## Patentansprüche

1. Verwendung einer Kosmetikzusammensetzung, umfassend: i) ein Organosiloxanharz in einem Betrag von 0,3 Gew.-% bis 10 Gew.-% der Zusammensetzung, um einen Haarerscheinungsbild reduzierenden Effekt auf der Haut bereitzustellen, und ii) einen dermatologisch akzeptablen Träger zum Reduzieren des Haarerscheinungsbildes in der Haut.

2. Verwendung nach Anspruch 1, wobei die Zusammensetzung ferner eine Haarwachstumsregulierungsverbindung umfasst, und wobei die Haarwachstumsregulierungsverbindung aus der Gruppe ausgewählt wird, die aus Butylhydroxytoluol, Butylhydroxyanisol, Hexamidinverbindungen, Cetylpyridiniumchlorid, Grüner Tee-Catechinen, Phytosterol, Malol, Difluormethylornithin, Alpha-Methylornithin, 1,8-Diaminooctan, Bathocuproin, Alanosin, Arginin, von Sojapflanzen abgeleiteten Extrakten, Zimtsäure, Cinnamylmethylanthranilat, Mannosamin und Salzen, Derivaten und Mischungen von irgendwelchen der Vorstehenden besteht.

3. Verwendung nach Anspruch 1 oder 2, wobei die Haarwachstumsregulierungsverbindung ausgewählt wird aus der Gruppe bestehend aus Butylhydroxytoluol, Butylhydroxyanisol, Hexamidin, Cetylpyridiniumchlorid, Malol und Kombinationen davon.

4. Verwendung nach einem der Ansprüche 1 bis 3, wobei die Zusammensetzung ferner ein Polyol umfasst, und wobei das Polyol im Bereich von 5 Gew.-% bis 50 Gew.-% der Zusammensetzung liegt.

5. Verwendung nach einem der Ansprüche 1 bis 4, wobei die Zusammensetzung Wasser im Bereich von 30 Gew.-% bis 80 Gew.-% der Zusammensetzung umfasst.

6. Verwendung nach einem der Ansprüche 1 bis 5, wobei die Zusammensetzung ein Dauer-Make-Up-Produkt ist, das ferner ein Pigment und mindestens einen Hautwirkstoff umfasst.

7. Verwendung nach einem der vorstehenden Ansprüche, wobei die Zusammensetzung entlang der Richtung des Haarwachstums auf dem Bereich der Haut aufgebracht wird, in dem eine Haarerscheinungsbildreduzierung gewünscht wird.

8. Verwendung nach Anspruch 7, wobei eine zweite Zusammensetzung auf der Haut aufgebracht wird, die mindestens eine Haarwachstumsregulierungsverbindung und einen dermatologisch akzeptablen Träger umfasst; und wobei sich die ersten und zweiten Zusammensetzungen voneinander in Form ihrer Zusammensetzungen unterscheiden.

9. Verwendung nach Anspruch 8, wobei die Haarwachstumsregulierungsverbindung der zweiten Zusammensetzung ausgewählt wird aus der Gruppe bestehend aus Butylhydroxytoluol, Butylhydroxyanisol, Hexamidin, Cetylpyridiniumchlorid, Malol und Kombinationen davon.

## Revendications

1. Utilisation d'une composition cosmétique comprenant : i) une résine d'organosiloxane en une quantité de 0,3 % à 10 % en poids de la composition pour fournir un effet de réduction de l'apparence des poils sur la peau, et ii) un véhicule acceptable dermatologiquement pour réduire l'apparence des poils dans la peau.

2. Utilisation selon la revendication 1, dans laquelle ladite composition comprend en outre un composé régulateur de croissance des cheveux, et dans laquelle ledit composé régulateur de croissance des cheveux est choisi parmi le groupe constitué d'hydroxytoluène butylé, d'hydroxyanisole butylé, de composés d'hexamidine, de chlorure de cétylpyridinium, de catéchines de thé vert, de phytostérols, d'acide ursolique, de difluorométhylornithine, d'alpha-méthylornithine, de 1,8-diaminooctane, de bathocuprione, d'alanosine, d'arginine, d'extraits dérivés de plantes de soja, d'acide cinnamique, d'anthranilate de cinnamyle, de mannosamine, et de sels, dérivés et mélanges de l'un de ces éléments.

3. Utilisation selon la revendication 1 ou 2, dans laquelle ledit composé régulateur de croissance des cheveux est choisi dans le groupe constitué d'hydroxytoluène butylé, d'hydroxyanisole butylé, d'hexamidine, de chlorure de cétylpyridinium, d'acide ursolique, et leurs combinaisons.

4. Utilisation selon l'une quelconque des revendications 1 à 3, dans laquelle ladite composition comprend en outre un polyol, et dans laquelle ledit polyol se situe dans la plage de 5 % à 50 % en poids de la composition.

5. Utilisation selon l'une quelconque des revendications 1 à 4, dans laquelle ladite composition comprend de l'eau dans la plage de 30 % à 80 % en poids de la composition.

6. Utilisation selon l'une quelconque des revendications 1 à 5, dans laquelle ladite composition est un produit de maquillage sans rinçage qui comprend en outre un pigment et au moins un agent actif pour la peau.

7. Utilisation selon l'une quelconque des revendications précédentes, dans laquelle ladite composition est appliquée dans le sens de la croissance des poils sur la zone de la peau où la réduction de l'apparence des poils est souhaitée.

8. Utilisation selon la revendication 7, dans laquelle une deuxième composition est appliquée sur la peau, qui comprend au moins un composé régulateur de croissance des poils et un véhicule acceptable dermatologiquement, et dans laquelle les première et deuxième compositions diffèrent l'une de l'autre par leurs compositions.

9. Utilisation selon la revendication 8, dans laquelle le composé régulateur de croissance des cheveux de la deuxième composition est choisi dans le groupe constitué par l'hydroxytoluène butylé, l'hydroxyanisole butylé, l'hexamidine, le chlorure de cétylpyridinium, l'acide ursolique et leurs combinaisons.
